# EUROPEAN PATENT APPLICATION

(11) **EP 3 594 889 A1**
(43) Date of publication of application: **15.01.2020**
(21) Application number: 17899774.8
(22) Date of filing: 08.03.2017
(51) Int. Cl.: G06Q 30/06, A41H 1/00, A61B 5/107, A61B 5/00, G06T 1/00

(54) **METHOD FOR TAKING HUMAN CHEST MEASUREMENT AND METHOD FOR SELECTING AND PROVIDING BRA SIZE**

(71) Applicant: Xiamen Brana Design Co., Ltd, Xiamen, Fujian 361024 (CN)
(72) Inventor: WANG, Zhongtang, Xiamen, Fujian 361024 (CN)
(74) Representative: Meyer, Thorsten
(86) International application number: PCT/CN2017/075926
(87) International publication number: WO 2018/161268

(57) **Abstract**

Disclosed is a method for method for determining a bra size, by uploading chest parameters to a server, analyzing the chest parameters, matching the chest parameters with data of a database, generating a bra size, and sending the bra size to the intelligent terminal, precise bra size determination is realized; and the bra size includes a band length, a bra cup size, a breast width and a breast convex height, which make it convenient for users to select well-fitted bras.

## Description

### FIELD OF THE INVENTION

The present invention relates to a method for determining a bra size.

### BACKGROUND OF THE INVENTION

Industrial mass production of bra has been promoted and costumers' needs have been largely fulfilled since bra sizes came out in 1935. Bra sizes simplify the measurement and calculation of chest measurements, so as to reduce bra cup sizes and patterns. However, female breast varies between individuals and suffers from gradual change, which makes it difficult to take accurate measurements, and consequently wrong bra size is selected.

Previous statistics showed that 80% of women wore incorrectly sized bras. The figure seems exaggerated since it is assumed that woman wearing bra everyday should have found a bra fitting for herself. But actually, most women were or are being confused about how to select a correctly sized bra during any stage of puberty growth period, adolescence, middle age and old age.

Bra band is made of elastic material and its length is easy to be determined. The user can easily adjust the band length as per personal preference. However, it is common that a bra cup fails to fit the user's breast, main reasons for which are listed as follows.
1) Shape of female breast varies between individuals. One bra size fitting a breast in hemispheric shape or teardrop shape may not always fit a breast in round shape though the breasts in all shapes are of similar volume. For the breast in round shape, partial breast tissue is out of coverage of the bra cup, so the bra cup fails to wrap the partial breast issue and gaps at the top; for breast in tubular shape or with noticeable sagging, the breast tissue is squeezed by the bra, which may induce breast disease.
2) A breast asymmetry due to different breast sizes occurs, wherein a breast asymmetry of this kind is generally caused by random growth pattern during breast development, genetic factors, or either breast removed partially or completely due to disease. In this case, the bra cup size is determined by average values of measurements of both breasts, as a result, one bra cup squeezes the breast, whereas the other bra cup gaps.
3) A breast asymmetry due to different breast convex heights occurs, in this case, the measurements taken intend to be larger than usual, then the bra cup fails to cover the breast fully.
4) Middle-aged or old-aged women are generally suffering from breast sagging in various amount, in this case, the measurements taken and the bra cup size are prone to be smaller than usual, therefore partial breast tissue extends toward the armpit and heaps up to form a tail of Spence.
5) Breast's position on a chest wall differs. A breast is usually situated on the chest wall between the second and the sixth ribs. Given the same volume, a breast in an upper position may look large and is more involved with the pectoralis major, while a breast in a lower position may look small and is less involved with the pectoralis major.
6) Bra size differs between brands. Bra size inconsistency not only lies between European and Japanese brands, but also between manufacturers. And it is not always convenient to try on a close-fitting bra before making a purchase. All these lower the chance to select a bra of right size.
7) The existing bra sizes and bra cup sizes determined by measurements and 3D data of breasts of a certain number of individuals are not enough for fitting for all women.

To sum up, the simple criterion for bra size determination used since 1935 is to blame for women's failure in selecting well-fitted bras. Bra manufacturers are willing yet unable to produce bras of more sizes as there is no better bra sizing criterion for reference.

Above all, it is necessary to provide a method for determining a bra size to improve the criterion and realize precise bra size determination, and make it convenient for users to select well-fitted bras.

### SUMMARY OF THE INVENTION

The present invention aims at providing a method for determining a bra size to realize precise bra size determination and make it convenient for users to select well-fitted bras.

To achieve the goals, the technical solutions of the present invention are described as follows.

A method for determining a bra size, comprising steps of:
uploading chest parameters to a server by a user,
analyzing the chest parameters by the server,
matching the chest parameters with data of a database by the server, and
generating a bra size;
wherein the chest parameters comprise an upper chest circumference, a lower chest circumference, an underarm chest circumference, an underarm skin soft tissue thickness, a breast arc length, a breast width, a breast convex height, a breast shape type, a body mass index and a breast softness type;
the data of the database is consisted of collected underarm skin soft tissue thickness data, breast shape type data, body mass index data and breast softness type dataof individuals;
the bra size is represented by Lxy (z1:z2) Rxy (z1:z2), wherein L represents a left breast, R represents a right breast, x is a band length, y is a bra cup size, z1 is a correctional breast width, and z2 is a correctional breast convex height;
the band length is derived from the lower chest circumference, the correctional breast width is derived from the breast width and is generated from the database, the correctional breast convex height is derived from the breast convex height and is generated from the database, and the bra cup size is determined jointly by the upper chest circumference, the lower chest circumference and the breast arc length and is generated from the database;
wherein the chest parameters further comprise a 2D or 3D breast image;
the method for determining a bra size further comprises a step as follows:
   when analyzing the chest parameters by the server, identifying, sharpening and cropping the 2D or 3D breast image,
   comparing the 2D or 3D breast image with the breast shape type and the body mass index uploaded by the user to judge an accuracy of the chest parameters, and
   revising the chest parameters.
wherein the method for determining a bra size specifically comprises steps as follows:
   step 1: measuring a breast sagging parameter, and uploading the breast sagging parameter to the server via an intelligent terminal by the user;
wherein the breast sagging parameter is a sagging or a non-sagging; if a nipple horizontal line is above an inframammary fold, the non-sagging is identified; if the nipple horizontal line is beneath or overlapped with the inframammary fold, the sagging is identified;
   step 2: sending methods for measuring the chest parameters to the user based on the breast sagging parameter;
wherein if non-sagging is identified,
the upper chest circumference is a length measured by placing a soft ruler on the nipples or on the peaks of the breasts, and wrapping the soft ruler around a torso in a standing position;
the lower chest circumference is a length measured by placing the soft ruler on the inframammary fold, and wrapping the soft ruler around the torso in a standing position;
the underarm chest circumference is a length measured by placing the soft ruler on lower armpits, and wrapping the soft ruler around the torso in a standing position and with the user's arms resting on the waist;
the underarm skin soft tissue thickness is a distance between a thumb and an index finger measured by using the thumb and the index finger to pinch the skin surface and subcutaneous soft tissue on an underarm area where a midaxillary line and the nipple horizontal line cross;
a method for measuring the breast arc length and the breast width is that pushing the breast inward toward a center with a palm to form a tissue dividing line between the breast and an anterior chest wall, and pushing the breast outward with the palm to form another tissue dividing line between the breast and a lateral chest wall; therefore, the nipple horizontal line and the tissue dividing line between the breast and the anterior chest wall cross and define an intersection point, the nipple horizontal line and the tissue dividing line between the breast and the lateral chest wall cross and define another intersection point; the breast arc length equals a distance spanning both intersection points, while the breast width equals a straight-line distance between both intersection points;
the breast convex height is a distance between a nipple base and a chest wall measured in a position that the breast is slightly pulled forward;
the breast shape type is selected from a round type, a hemispheric type, a teardrop type, a conical type, a tubular type and a beak-like type;
the breast softness is semiquantitatively graded as extra soft, soft, medium soft or firm;
the body mass index is calculated by dividing body mass expressed in kilogram by the square of a body height expressed in meter, and categorizes the user's body type as underweight, normal, overweight or obese;
wherein if sagging is identified,
the breasts are lifted to measure the upper chest circumference, the lower chest circumference, the underarm chest circumference, the breast arc length and the breast width;
   step 3: uploading the chest parameters to the server via the intelligent terminal;
wherein the chest parameters are average values of multiple measurements;
   step 4: analyzing the chest parameters, matching the chest parameters with the data of the database, and generating the bra size;
the data of the database is consisted of collected underarm skin soft tissue thickness data, breast shape type data, body mass index data and breast softness type dataof individuals;
analyzing the underarm skin soft tissue thickness, the breast shape type, the body mass index and the breast softness type uploaded by the user, matching them with the data of the database, determining the bra cup size according to a matching result, and generating the bra size;
   step 5: sending the bra size to the intelligent terminal by the server;
wherein the bra size is represented by Lxy (z1:z2) Rxy (z1:z2), L represents the left breast, R represents the right breast, x is the band length, y is the bra cup size, z1 is the correctional breast width, and z2 is the correctional breast convex height;
   step 6: purchasing a bra or ordering a custom-made bra according to the generated bra size, and uploading feedback information on a wearing experience to the server via the intelligent terminal; and
   step 7: optimizing the database by revising the chest parameters according to the feedback information and the chest parameters uploaded by the user.

With the method for determining a bra size provided in the present invention, by uploading chest parameters to a server, analyzing the chest parameters, matching the chest parameters with data of a database, generating a bra size, and sending the bra size to the intelligent terminal, precise bra size determination is realized; wherein the bra size includes the band length, the bra cup size, the breast width and the breast convex height, which make it convenient for users to select well-fitted bras.

### BRIEF DESCRIPTION OF THE DRAWINGS

Fig. 1 is a schematic drawing showing measurement of an upper chest circumference, a lower chest circumference and an underarm chest circumference in a standing position;
Fig. 2 is a schematic drawing showing measurement of an underarm skin soft tissue thickness in the standing position;
Fig. 3A is a first schematic drawing showing measurement of a breast arc length and a breast width;
Fig. 3B is a second schematic drawing showing measurement of the breast arc length and the breast width;
Fig. 3C is a schematic drawing showing measurement of the breast arc length;
Fig. 3D is a schematic drawing showing measurement of the breast width;
Fig. 4 is a schematic drawing showing measurement of the upper chest circumference in a lying position;
Fig. 5 is a schematic drawing showing measurement of the breast arc length in the lying position;
Fig. 6 is a schematic drawing showing measurement of an upper chest circumference of a saggy breast;
Fig. 7 is a schematic drawing showing measurement of a breast arc length of the saggy breast;
Fig. 8 shows various types of breast shape;
Fig. 9A shows a first type of breast asymmetry;
Fig. 9B shows a second type of breast asymmetry;
Fig. 9C shows a third type of breast asymmetry;
Fig. 9D shows a fourth type of breast asymmetry;
Fig. 9E shows a fifth type of breast asymmetry;
Fig. 10A shows a first type of breast sagging;
Fig. 10B shows a second type of breast sagging;
Fig. 10C shows a third type of breast sagging;
Fig. 10D shows a fourth type of breast sagging;
Fig. 11 is a schematic drawing showing measurement of a breast convex height;
Fig. 12 is a flow chart of determining a bra size;
Fig. 13 is a flow chart of chest parameters processing.

### DETAILED DESCRIPTION OF ILLUSTRATED EMBODIMENTS

In order to describe the technical content, structural features, objectives and effects achieved, the present invention will be further explained below in detail with reference to embodiments and figures.

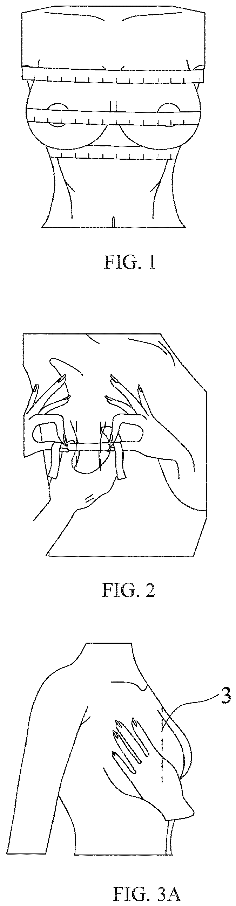

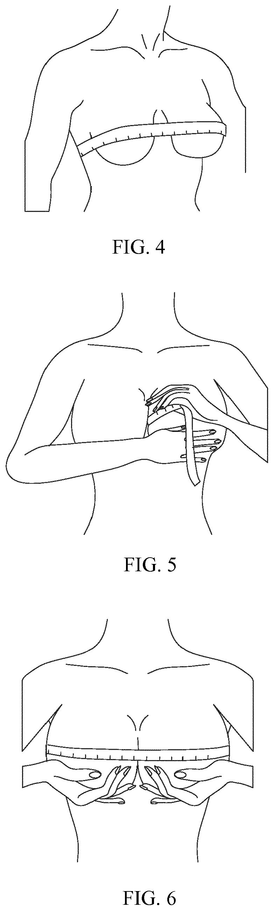

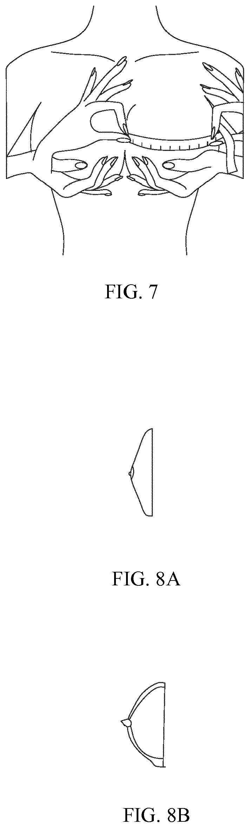

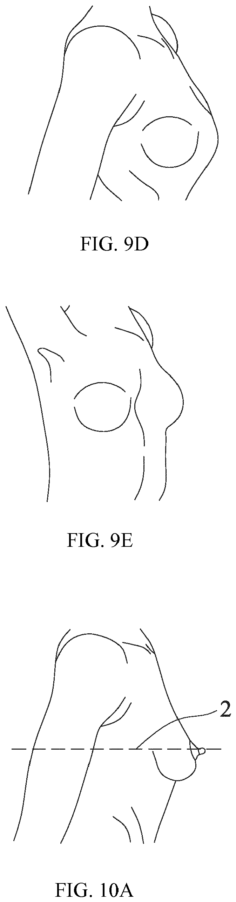

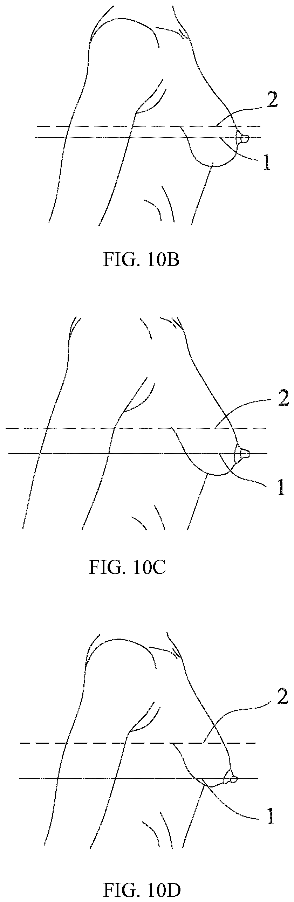

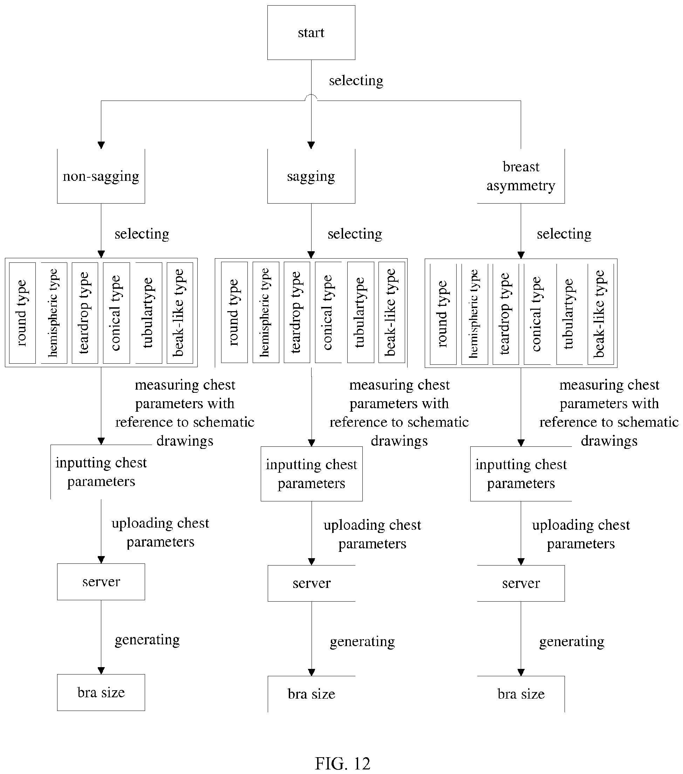

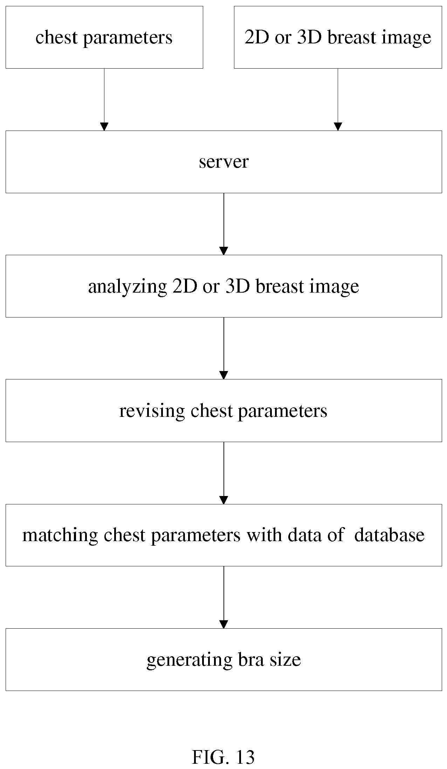

Referring to Figs. 1 to 13, the present invention discloses a method for determining a bra size. By uploading chest parameters to a server, analyzing the chest parameters, matching the chest parameters with data of a database, generating a bra size, and sending the bra size to the intelligent terminal, precise bra size determination is realized; wherein the bra size includes the band length, the bra cup size, the breast width and the breast convex height, which make it convenient for users to select well-fitted bras. The method for determining a bra size comprises steps as follows:
step 1: measuring a breast sagging parameter, and uploading the breast sagging parameter to the server via an intelligent terminal by the user;
   referring to Figs. 10A to 10D, the breast sagging parameter is a sagging or a non-sagging; if a nipple horizontal line **1** is above an inframammary fold **2,** the non-sagging is identified; if the nipple horizontal line **1** is beneath or overlapped with the inframammary fold **2,** the sagging is identified;
step 2: sending methods for measuring the chest parameters to the user based on the breast sagging parameter; when necessary, sending schematic drawings to the user to better guide through the measurement;
   wherein the chest parameters need to be measured by the user comprise an upper chest circumference, a lower chest circumference, an underarm chest circumference, an underarm skin soft tissue thickness, a breast arc length, a breast width, a breast convex height, a breast shape type, a body mass index, a breast softness type and a 2D or 3D breast image;
   when measuring the chest parameters, the user shall undress her upper body and perform one of the postures, e.g. standing straight with shoulders and ankles in line, standing with upper body forward, or lying flat; users free from breast sagging can refer to Figs. 1 to 5 to take the measurements by herself or with assistance of others, while users suffering from breast sagging as shown in Figs. 10A to 10D shall lift the breasts to a position above the inframammary fold with reference to Figs. 6 and 7 and take the measurements with assistance of others.

Referring to Fig. 1, the upper chest circumference is a length measured by placing a soft ruler on the nipples or on the peaks of the breasts, and wrapping the soft ruler around a torso in a standing position;
the lower chest circumference is a length measured by placing the soft ruler on the inframammary fold, and wrapping the soft ruler around the torso in a standing position;
the underarm chest circumference is a length measured by placing the soft ruler on lower armpits, and wrapping the soft ruler around the torso in a standing position and with the user's arms resting on the waist;
wherein the underarm chest circumference is configured to estimate the development of the chest muscle and serves as a parameter for producing full cup bra;
wherein the upper chest circumference, the lower chest circumference or the underarm chest circumference reaches a maximum value after meal or after breathing in to a greatest extent, and reaches a minimum value under fasting condition or after breathing out to a greatest extent; in practice, value of the upper chest circumference, the lower chest circumference or the underarm chest circumference measured during two-hour postpartum and under even breathing shall prevail.

Fig. 2 is a schematic drawing showing measurement of the underarm skin soft tissue thickness; the underarm skin soft tissue thickness is a distance between a thumb and an index finger measured by using the thumb and the index finger to pinch the skin surface and subcutaneous soft tissue on an underarm area where a midaxillary line and the nipple horizontal line cross;

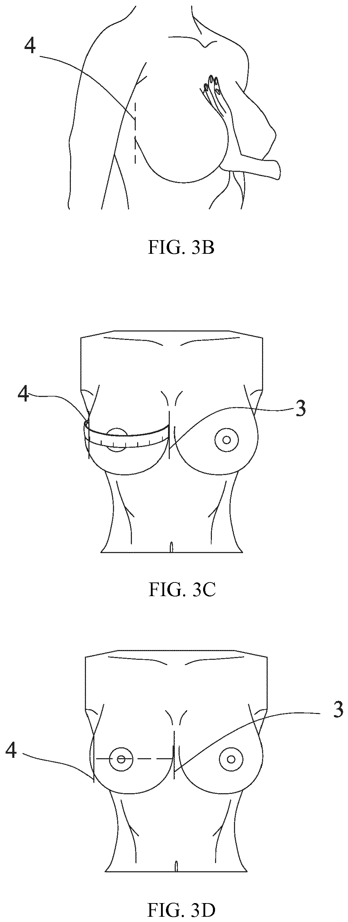

Figs. 3A to 3D are schematic drawings showing measurement of the breast arc length and the breast width; pushing the breast inward toward a center with a palm to form a tissue dividing line **3** between the breast and an anterior chest wall, and pushing the breast outward with the palm to form another tissue dividing line **4** between the breast and a lateral chest wall; therefore, the nipple horizontal line and the tissue dividing line **3** between the breast and the anterior chest wall cross and define an intersection point, the nipple horizontal line and the tissue dividing line **4** between the breast and the lateral chest wall cross and define another intersection point; the breast arc length equals a distance spanning both intersection points, while the breast width equals a straight-line distance between both intersection points;
by combining the breast arc length, the breast width and the breast shape type, a breast volume can be approximately calculated; according to the breast volume, it is possible to evaluate the effects of breast augmentation or reduction and weight losing or gaining, or monitor the growth of breast tumour or abscess, or guide the user in bra selection;
wherein the breast arc length and the breast width can be measured with a daily soft ruler.

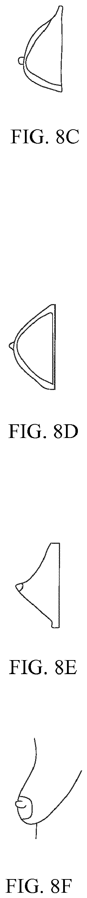

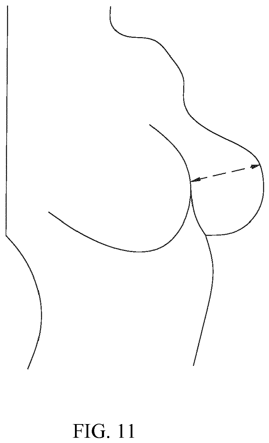

Fig. 11 is a schematic drawing showing measurement of breast convex height; the breast convex height is a distance between a nipple base and a chest wall measured in a position that the breast is slightly pulled forward;
Referring to Fig. 8, the breast shape type is selected from a round type, a hemispheric type, a teardrop type, a conical type, a tubular type and a beak-like type;
wherein the breast shape type can be confirmed by the user.

The breast softness is semiquantitatively graded as extra soft, soft, medium soft or firm.

The body mass index is calculated by dividing body mass expressed in kilogram by the square of a body height expressed in meter, and categorizes the user's body type as underweight, normal, overweight or obese.

Step 2: uploading the chest parameters to the server via the intelligent terminal;
specifically, inputting the chest parameters in the intelligent terminal such as a smart phone, a computer, etc., and sending the chest parameters to the server via the intelligent terminal; wherein the chest parameters can be average values of multiple measurements, in a practice instance, measuring the chest parameters three times and uploading average values of three measurements to the server via the intelligent terminal, or alternatively, uploading values of the three measurements to the server via the intelligent terminal and letting the server calculate the average values.

Step 3: analyzing the chest parameters, matching the chest parameters with the data of the database, and generating the bra size;
the data of the database is consisted of collected underarm skin soft tissue thickness data, breast shape type data, body mass index data and breast softness type dataof individuals;
wherein the database is consisted of various chest parameter data of individuals collected from literature and the chest parameters uploaded by the user; reliability of the data collected from literature is evaluated by authoritativeness of the literature, and size and nationality diversity of individual samples.

The bra size is consisted of the band length, the bra cup size, the breast width and the breast convex height, wherein the band length is derived from the lower chest circumference, the bra cup size is determined jointly by the upper chest circumference, the lower chest circumference and the breast arc length, the correctional breast width is derived from the breast width and is generated from the database; the correctional breast convex height is derived from the breast convex height and is generated from the database;
wherein the bra cup size is represented by letters A through I and increases in steps of 1.27 centimeters or 0.5 inch instead of 2.54 centimeters or 1 inch, and half size is available.
analyzing the underarm skin soft tissue thickness, the breast shape type, the body mass index and the breast softness type uploaded by the user; after the 2D or 3D breast image is uploaded, identifying, sharpening and cropping the 2D or 3D breast image, comparing the 2D or 3D breast image with the chest parameters uploaded by the user, such as the breast shape type, the body mass index, the breast sagging parameter, amount of breast sagging or a breast malformation parameter, so as to judge the accuracy of the chest parameters, and revising the chest parameters.
after the analysis, matching the underarm skin soft tissue thickness, the breast shape type, the body mass index and the breast softness type with the data of the database, determining the bra cup size according to a matching result, and generating the bra size;
wherein as the underarm skin soft tissue thickness advertises a thickness of soft tissue on a chest and a back, if the underarm skin soft tissue thickness does not exceed 1 inch, applying regular calculation for determining the band length and the bra cup size; if the underarm skin soft tissue thickness exceeds 1 inch, decreasing the band length by one inch and increasing the bra cup size by half size for one extra inch of the underarm skin soft tissue thickness. Provided wearing comfort is guaranteed, an increase of bra cup size is to improve a wrapping effect and to reduce excessive breast movement during participation in sport.

The breast softness is semiquantitatively graded as extra soft, soft, medium soft or firm, wherein the breast softness is determined by a tissue composition ratio of the breast. A breast with more fatty tissue is relatively soft, while a breast with more glandular tissue is relatively firm. For the breast with more fatty tissue, the bra cup size can be a bit smaller than a regular size; for the breast with more glandular tissue, the bra cup size can be a bit larger than a regular size. Provided the wearing comfort is guaranteed, the increase of the bra cup size is to improve the wrapping effect and to reduce excessive breast movement during participation in sport.

Step 4: sending the bra size to the intelligent terminal by the server;
further, sending reminding message along with the the bra size to the user, wherein the reminding message is to warn against an abnormal result, to remind the user to remeasure the chest parameters, or advise the user to order a customer-made bra or to ask for advice from a bra designer or a plastic surgery expert.

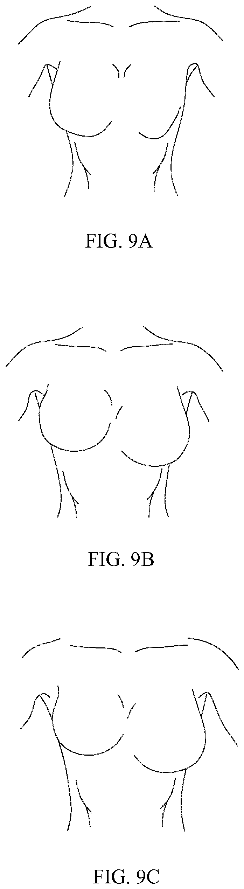

Referring to Fig. 9A to 9E, the bra size is represented by Lxy (z1:z2) Rxy (z1:z2), wherein L represents a left breast, R represents a right breast, x is a band length, y is a bra cup size, z1 is the correctional breast width, and z2 is the correctional breast convex height;
if a breast asymmetry occurs, the bra size is consisted of a left size and a right size, wherein the sizes can be expressed in either of centimeter or inch;
for instance, 75B (15:7.5) / 32B (6:3) means that the size and shape of the left breast is similar to those of the right breast, wherein 75B (15:7.5) is expressed in centimeter and indicates a size of 75 cm band length, B cup size, 15 cm breast width and 7.5 cm breast convex height, while 32B (6:3) is expressed in inch and indicates a seize of 32 inch band length, B cup size, 6 inch breast width and 3 inch breast convex height.

L 75A (12.5:5.0) R 75D (17.5 : 10.0) / L 32A (5:2) R 32D (7:4) means the breast asymmetry occurs, wherein L 75A (12.5:5.0) R 75D (17.5 :10.0) is expressed in centimeter and indicates a left breast size of 75 cm band length, A cup size, 12.5 cm breast width and 5.0 cm breast convex height, and a right breast size of 75 cm band length, D cup size, 17.5 cm breast width and 10.0 cm breast convex height, while L 32A (5:2) R 32D (7:4) is expressed in inch and indicates a left breast size of 32 inch band length, A cup size, 5 inch breast width and 2 inch breast convex height, and a right breast size of 32 inch band length, D cup size, 7 inch breast width and 4 inch breast convex height.

72.5B+ (12.5:7.5) / 31B+ (5:3) means that the size and shape of the left breast is similar to those of the right breast; the band length is 72.5 cm (31 inch); the bra cup size is B+, which represents a bra cup lager than B cup yet smaller than C cup, the breast width is 12.5 cm (5 inch), and the breast convex height is 7.5 cm (3 inch).

Step 5: purchasing a bra or ordering a custom-made bra according to the generated bra size, and uploading feedback information on a wearing experience, such as feeling that the bra size is well-fitted, too large or too small, to the server via the intelligent terminal.

Step 6: optimizing the database according to the feedback information;
specifically, optimizing the database by adding or deleting the chest parameters, or amending weights of the chest parameters based on the feedback information and the chest parameters uploaded by the user and with the aid of artificial intelligence, wherein the artificial intelligence is selected from machine learning, deep machine learning and multi-tasking learning;
wherein optimizing the database is to layer the database to multiple hierarchies as the data increases;
adding or deleting the chest parameters is to add or delete the chest parameters according to use condition;
amending the weights of the chest parameter is to adjust their influence of the chest parameters on determining the bra size according to use condition.

The above disclosed condiments are merely preferred embodiments of the present invention, and certainly do not limit the scope of the present invention. Therefore, equivalent changes made according to the scope of the present invention still fall within the scope of the present invention.

## Claims

1. A method for determining a bra size, comprising steps of:
uploading chest parameters to a server by a user,
analyzing the chest parameters by the server,
matching the chest parameters with data of a database by the server, and
generating a bra size;
wherein the chest parameters comprise an upper chest circumference, a lower chest circumference, an underarm chest circumference, an underarm skin soft tissue thickness, a breast arc length, a breast width, a breast convex height, a breast shape type, a body mass index and a breast softness type;
the data of the database is consisted of collected underarm skin soft tissue thickness data, breast shape type data, body mass index data and breast softness type dataof individuals;
the bra size is represented by Lxy (z1:z2) Rxy (z1:z2), wherein L represents a left breast, R represents a right breast, x is a band length, y is a bra cup size, z1 is a correctional breast width, and z2 is a correctional breast convex height;
the band length is derived from the lower chest circumference, the correctional breast width is derived from the breast width and is generated from the database, the correctional breast convex height is derived from the breast convex height and is generated from the database, and the bra cup size is determined jointly by the upper chest circumference, the lower chest circumference and the breast arc length and is generated from the database.

2. The method for determining a bra size according to claim 1, wherein the chest parameters further comprise a 2D or 3D breast image;
the method further comprises a step as follows:
when analyzing the chest parameters by the server, identifying, sharpening and cropping the 2D or 3D breast image,
comparing the 2D or 3D breast image with the breast shape type and the body mass index uploaded by the user to judge an accuracy of the chest parameters, and
revising the chest parameters.

3. The method for determining a bra size according to claim 1 or 2, wherein the method comprises steps as follows:
step 1: measuring a breast sagging parameter, and uploading the breast sagging parameter to the server via an intelligent terminal by the user;
wherein the breast sagging parameter is a sagging or a non-sagging; if a nipple horizontal line (1) is above an inframammary fold (2), the non-sagging is identified; if the nipple horizontal line (1) is beneath or overlapped with the inframammary fold (2), the sagging is identified;
step 2: sending methods for measuring the chest parameters to the user based on the breast sagging parameter;
wherein the chest parameters comprise the upper chest circumference, the lower chest circumference, the underarm chest circumference, the underarm skin soft tissue thickness, the breast arc length, the breast width, the breast convex height, the breast shape type, the body mass index and the breast softness type;
wherein if non-sagging is identified,
the upper chest circumference is a length measured by placing a soft ruler on the nipples or on the peaks of the breasts, and wrapping the soft ruler around a torso in a standing position;
the lower chest circumference is a length measured by placing the soft ruler on the inframammary fold (2), and wrapping the soft ruler around the torso in a standing position;
the underarm chest circumference is a length measured by placing the soft ruler on lower armpits, and wrapping the soft ruler around the torso in a standing position and with the user's arms resting on the waist;
the underarm skin soft tissue thickness is a distance between a thumb and an index finger measured by using the thumb and the index finger to pinch the skin surface and subcutaneous soft tissue on an underarm area where a midaxillary line and the nipple horizontal line (1) cross;
a method for measuring the breast arc length and the breast width is that pushing the breast inward toward a center with a palm to form a tissue dividing line (3) between the breast and an anterior chest wall, and pushing the breast outward with the palm to form another tissue dividing line (4) between the breast and a lateral chest wall; therefore, the nipple horizontal line (1) and the tissue dividing line (3) between the breast and the anterior chest wall cross and define an intersection point, the nipple horizontal line (1) and the tissue dividing line (4) between the breast and the lateral chest wall cross and define another intersection point; the breast arc length equals a distance spanning both intersection points, while the breast width equals a straight-line distance between both intersection points;
the breast convex height is a distance between a nipple base and a chest wall measured in a position that the breast is slightly pulled forward;
the breast shape type is selected from a round type, a hemispheric type, a teardrop type, a conical type, a tubular type and a beak-like type;
the breast softness is semiquantitatively graded as extra soft, soft, medium soft or firm;
the body mass index is calculated by dividing body mass expressed in kilogram by the square of a body height expressed in meter, and categorizes the user's body type as underweight, normal, overweight or obese;
wherein if sagging is identified,
the breasts are lifted to measure the upper chest circumference, the lower chest circumference, the underarm chest circumference, the breast arc length and the breast width;
step 3: uploading the chest parameters to the server via the intelligent terminal;
wherein the chest parameters comprise the upper chest circumference, the lower chest circumference, the underarm chest circumference, the underarm skin soft tissue thickness, the breast arc length, the breast width, the breast convex height, the breast shape type, the body mass index and the breast softness type;
wherein the chest parameters are average values of multiple measurements;
step 4: analyzing the chest parameters, matching the chest parameters with the data of the database, and generating the bra size;
the data of the database is consisted of collected underarm skin soft tissue thickness data, breast shape type data, body mass index data and breast softness type dataof individuals;
analyzing the underarm skin soft tissue thickness, the breast shape type, the body mass index and the breast softness type uploaded by the user, matching them with the data of the database, determining the bra cup size according to a matching result, and generating the bra size; and
step 5: sending the bra size to the intelligent terminal by the server;
wherein the bra size is represented by Lxy (z1:z2) Rxy (z1:z2), L represents the left breast, R represents the right breast, x is the band length, y is the bra cup size, z1 is the correctional breast width, and z2 is the correctional breast convex height.

4. The method for determining a bra size according to claim 3, wherein the method further comprises steps as follows:
step 6: purchasing a bra or ordering a custom-made bra according to the generated bra size, and uploading feedback information on a wearing experience to the server via the intelligent terminal; and
step 7: optimizing the database by revising the chest parameters according to the feedback information and the chest parameters uploaded by the user.
